# EUROPEAN PATENT APPLICATION

(11) **EP 3 874 994 A1**
(43) Date of publication of application: **08.09.2021**
(21) Application number: 21157048.6
(22) Date of filing: 15.02.2021
(51) Int. Cl.: A47G 9/08, A61M 21/02, A41B 13/06

(54) **SLEEPING BAG FOR THERAPEUTIC TREATMENT**

(30) Priority: 05.03.2020 EP 20161174
(71) Applicant: Protac A/S, 8660 Skanderborg (DK)
(72) Inventor: CHRISTIANSEN, Pia Riishøj, 8660 Skanderborg (DK); PEDERSEN, Birthe Søndergaard, 8660 Skanderborg (DK)
(74) Representative: Zacco Denmark A/S

(57) **Abstract**

Disclosed herein is a sleeping bag for therapeutic treatment comprising a therapeutic material (150) which adapt to the body of the user and exert a sporadic pressure on the body of the user, wherein the therapeutic material (150) is selected from the group of:

-substantially spherical objects;

-fibre strips;

-granulate, such as granulate made from glass, polyethylene, polypropylene, silicone, peas, beans, lenses, or similar;

-substantially ring-formed objects;

-chains, e.g. polymeric and/or metal objects connected to form a chain.

## Description

### Background

It is known that persons who suffer from anxiety, unrest, or sensory disorders have difficulty in finding rest, sleeping, or relaxing. A reason for this may be that they have difficulty in feeling the boundaries of their own bodies, and they can need a physical object to focus on, preferably something that presses against the body and that they can bend and wrap around themselves. Especially for hyperactive, restless children or children with an ADHD problem or Autism/Asperger's syndrome, a physical object makes a great difference when calm, sleep, or insufficient relaxation is involved, but also children and adults with a general complaint of sleep problems, unrest, and angst can benefit from a simple and practical solution to relieve the complaint.

By pressure and deep pressure touch applied to the body, the user receives a feeling of safety, and it is believed that a number of bodily substances are released that have a calming effect, including oxytocin. If it is possible to follow the form of the body and to vary the pressure between certain points, this contributes to tactile and proprioceptive stimulation and can increase the positive result for the user.

It is known to use filled blankets for this purpose. Some variants show a blanket that contains plastic pellets or balls. These materials all have the common feature that they are based on essentially point-like objects. In order to keep the fillings from moving to the lowest point of the blanket during use, patches, preferably with a square shape, are often used. EP0729340, EP2983567 and EP2950768 are examples of blankets with spherical objects for therapeutic treatment.

### Summary

Disclosed herein is a sleeping bag for therapeutic treatment. The sleeping bag comprises a base part on which a user rests, and a cover part covering the user's body when positioned in the sleeping bag. The base part comprises an outer surface; an inner surface; an edge surface connecting peripheral edges of the surfaces; and a base filling between the outer surface and the inner surface.

Likewise, the cover part comprises an outer surface; an inner surface; an edge surface connecting peripheral edges of the surfaces, wherein at least a part of the body edge surfaces, are releasable connected for providing an easy user access to the sleeping bag.

The cover part further comprises a number of cover part sections with cover filling between the outer surface and the inner surface, the sections and cover filling including:
- a top section with a top section filling, the top section being positioned on a user's chest;
- a bottom section with a bottom section filling, the bottom section being positioned on the user's feet and possibly the lower part of the user's leg; and
- a middle section with a middle section filling, the middle section being positioned between the top section and the bottom section.

The base filling and the one or more cover filings comprises one or more soft material layers, and the middle section filling further comprises a therapeutic material which adapt to the body of the user and exert a sporadic pressure on the body of the user through the inner surface.

By section is meant a part of the cover part. A section is not necessarily separable from another section. A section may instead also refer to a portion or an area of the cover part. The border between the different sections may or may not be visible for the user by looking at the sleeping bag. The border between the different sections may or may not be detectible physically by a user touching/handling the cover part.

By soft material is meant a material resembling a filling material of a conventional blanket. By therapeutic material is meant a material, which exerts a therapeutic effect, e.g. weight effect, a sporadic effect or similar on the user's body.

By the above is obtained a sleeping bag, which encloses the entire body of the user, ensuring that the user cannot easily remove the sleeping bag during sleep. The sleeping bag may have a maximum width and a maximum length, where the maximum length is at least twice of that of the maximum width, it has a smaller size than the know blankets for therapeutic treatment. This provides for a sleeping bag, which has a lower weight and size making it easier to transport as compared to a regular blanket filled with therapeutic material. Further, the same therapeutic effect is obtained with the smaller sleeping bag as would be obtained from the much larger and heavier blankets, since the closed edges of the sleeping bag ensures that the therapeutic material stays on the user's body during use. Contrary with blanket, a much larger area filled with therapeutic material is required if the therapeutic effect is to be maintained during sleep, since a user nearly always manages to move him/herself and the blanket in relation to each other during sleep.

The sleeping bag additionally provides the user with an improved feeling of the boundaries of their body.

In one or more examples, the therapeutic material is positioned between an inner surface and an outer surface of the cover part and/or base part, e.g. such as between the inner surface and the outer surface of the middle section. The therapeutic material is arranged to adapt to the body of the user and exert a sporadic pressure on the body of the user through the inner surface.

In one or more examples, the base filling does not comprise the therapeutic material. This ensures that the layer on which the user is positioned is comfortable.

In one or more examples, the top section filling does not comprise the therapeutic material. This is advantageous when the user of the sleeping bag is e.g. a small child, where it may not be advice to position a heavy weight directly on the chest.

In one or more examples, the top section filling further comprises the therapeutic material. Alternatively, or in combination, the bottom section filling further comprises the therapeutic material. Thereby the user may obtain the therapeutic effect over a larger area of the body.

In one or more examples, at least a part of a bottom section abutting the border between the middle section and the bottom section may be provided with therapeutic material. This allows for the area of therapeutic material of the middle section to be contiguous with the area of therapeutic material of the bottom section.

In one or more examples, the bottom section may be provided with therapeutic material except for at least an area substantially extending over the feet of the user, when the sleeping bag is in use. In one or more examples, the entire extent of bottom section may be provided with therapeutic material.

In one or more examples, the one or more soft material layers, are selected from the group of:
- polymeric fibres, such as polyester fibres;
- hollow fibres, such as hollow polymeric fibres, such as hollow polyester fibres;
- plant fibres, such as fibres from corn, bamboo, viscose, jute, pineapple, seaweed, kapok, hemp;
- cupro fibres;
- wadding, such as polymeric wadding, such as polyester wadding;
- down.

By wadding is meant any fibrous or soft material for stuffing, padding, packing, etc., e.g. carded cotton in sheets.

By cupro fibres is meant cuprammonium rayon fibres. Cuprammonium rayon may be produced by making cellulose a soluble compound by combining it with copper and ammonia. The solution of this material in caustic soda is passed through the spinneret and the cellulose is regenerated in the hardening baths that remove the copper and ammonia and neutralize the caustic soda. Cuprammonium rayon is usually made in fine filaments that may be used to make textured fabrics. The fabric may also be known as cupro, cupra, or ammonia silk and these definitions are intended to be included in the definition of cupro fibres used herein.

In one or more examples, the sleeping bag comprises a first layer of fabric constituting the outer surfaces, and a second layer of fabric constituting the inner surfaces, wherein the soft material layers are positioned between the layers of fabric in both the cover part and the base part. The outer surface fabric layer of the cover part may be different from the outer fabric layer in the base part. Likewise, the inner surface fabric layer of the base part may be different from the inner surface fabric layer. Also, the inner fabric layers may differ from the outer fabric layers. Allowing for different material types of the fabric layers enables different material properties to be utilized for the cover part as compared to the base part and/or for the inner material layers as compared to the outer material layers.

In other examples, the fabric layers are made from the same fabric. This provides for a reduced fabrication cost, and for a visual uniformly appearing sleeping bag.

In one or more examples, the one or more soft material layers, comprises one or more layers of temperature stabilising and/or insulating material. By temperature stabilising material is meant a material, which can keep the body temperature of the user stable. Thereby, the temperature stabilising material may warm the user if the user is cold and cool the user if the user is too warm. This assists in providing the user with a pleasant and undisturbed sleep.

In one or more examples, the therapeutic material is positioned inside an elastic material. The elastic material ensures that the sleeping bag can be rolled up for easy transportation. By elastic material is meant a material, which can be stretched compared to its original form by at least 5-25% or such as by at least 5-50%, such as by at least 10-40% or such as by at least 10-30%. The stretching may be possible in all directions or only along some directions. An example of an elastic material is a jersey material. Another example of an elastic material is a tricot material and/or a woven material and/or a knit fabric, which may for example contain elastane.

In one or more examples, the one or more soft material layers comprises a first layer of nonwoven material and/or a second layer of nonwoven material. By non-woven fabric layer is meant a layer of fabric-like material made from staple fibre short and long fibres continuous long, bonded together by chemical, mechanical, heat or solvent treatment. The term is also understood to include fabrics, such as felt, which are neither woven nor knitted. The term also includes plant fibres as exemplified above.

In one or more examples, the elastic material is positioned between the layers of non-woven material. In one or more examples, the elastic material is positioned between a layer of non-woven material and the outer surface or the inner surface.

In one or more examples, the base part only comprises one layer of nonwoven material. The base part will normally not comprise therapeutic material why multiple layers of nonwoven materials are not required.

In the filling sections comprising the therapeutic material, the therapeutic material may be positioned between the first layer of nonwoven material and the second layer of nonwoven material. Often the elastic material will be found between the therapeutic material and the nonwoven layers.

In one or more examples, the one or more soft material layers comprises a hollow fibres layer of hollow fibres, such as hollow polymeric fibres, such as hollow polyester fibres. The hollow fibres provides an insulating effect. Further, the fibres in the base part provides for a soft base for the user to rest on.

In one or more examples, the hollow fibres layer, is positioned between a layer of nonwoven material and a fabric layer. In the cover part, the hollow fibres layer is normally positioned between the outer fabric layer and the first layer of non-woven material. In the base part, the hollow fibres layer is normally positioned between the inner fabric layer and the layer of nonwoven material.

In one or more examples, the one or more soft material layers comprises a layer kapok or down or a mixture thereof and the layer may be positioned between a layer of nonwoven material or the elastic material and a fabric layer. In the cover part, the down/kapok layer is normally positioned between the outer fabric layer and the first layer of non-woven material. In the base part, the down/kapok layer is normally positioned between the inner fabric layer and the layer of nonwoven material or the elastic material.

In one or more examples, the one or more soft material layers comprises a wadding layer such as polymeric wadding, such as polyester wadding. Hollow fibres, such as hollow polymeric fibres, such as hollow polyester fibres may be used as an alternative to the wadding. The wadding layer is normally positioned between the inner fabric layer and the layer of non-woven material. In one or more examples, the wadding layer is positioned between the two layers of nonwoven material.

In one or more examples, the therapeutic material may be made of recycled plastic materials, such as materials originating from fish nets or plastic bottles or other plastic items. The therapeutic may be made of natural or synthetic materials such as plastics or biomaterials such as plant-material or a combination thereof.

In one or more examples, the therapeutic material is selected from the group of:
- substantially spherical objects, e.g. polymeric and/or metal objects;
- fibre strips;
- granulate, e.g. glass or polymeric granulate;
- substantially ring-formed objects, e.g. glass or polymeric objects;
- chains, e.g. polymeric and/or metal objects connected to form a chain.

In one or more examples, the therapeutic material is selected from the group of:
- substantially spherical objects; such as objects made from metal (such as steel), glass, polyethylene, polypropylene, polystyrene, silicone, seaweed, hemp, pineapple, corn, peas, beans (such as soy beans), lenses, or similar;
- fibre strips;
- granulate, such as granulate made from metal (such as steel), glass, polyethylene, polypropylene, polystyrene, silicone, seaweed, hemp, pineapple, corn, peas, beans (such as soy beans), lenses, or similar;
- substantially ring-formed objects; such as objects made from metal (such as steel), glass, polyethylene, polypropylene, polystyrene, silicone, seaweed, hemp, pineapple, corn, peas, beans (such as soy beans), lenses, or similar;
- chains, e.g. polymeric and/or metal objects connected to form a chain, and/or chains made from metal (such as steel), glass, polyethylene, polypropylene, polystyrene, silicone, seaweed, hemp, pineapple, corn, peas, beans (such as soy beans), lenses, or similar.

The polymeric and/or metal objects may be metal objects with a plastic covering.

The therapeutic material may be positioned in a plurality of bags. Each bag may further have varying weights. Thus, the weight of a first bag may be different from the weight of a second bag. In this manner, different weights of e.g. spherical objects of granulate may be included as fillings at different positions in the sleeping bag depending on which body part(s) that sleeping bag is covering.

In one or more examples, a plurality of channels is formed between the outer surface and inner surface of at least the middle section in the cover part, by one or more joints.

The channels may be filled with the therapeutic material. The channels may run in the length-wise and/or width-wise direction of the sleeping bag. Other directional channels may alternatively be made in the cover part. In one or more examples, the channels are evenly distributed.

In one or more examples, the channels are formed in the elastic material covering the therapeutic material.

The one or more the joints may be a stitching. A stitching is considered a barrier of limited size. The combination of the channels with the stitching barrier prevent the substantially spherical objects from clumping together and obstructing the operation when a user wants to roll-up or pack up the sleeping bag, e.g. for transportation.

Each of the channels may be of a size that does not allow one substantially spherical object / one substantially ring-formed object / one bag with therapeutic material to pass another substantially spherical object / substantially ring-formed object / bag with therapeutic material, respectively, within the channel. By limiting the passage of the therapeutic material, a predefined distribution of the therapeutic material can be maintained during use or transportation of the sleeping bag. It is also avoided that the therapeutic material moves to one end of the sleeping bag if the user moves during sleep. Further, it is easier to regulate the applied pressure on a specific region of the body. Also, the thickness of the sleeping bag can be more uniform with a reduced thickness since two objects cannot be laying on top of each other. The reduced thickness also results in a sleeping bag with better temperature regulation properties.

In one or more examples, the therapeutic material in the middle section filling is positioned in the plurality of bags and the therapeutic material in the bottom section filling is positioned directly in the plurality of channels. The opposite scenario is also possible. In this manner, different types of therapeutic effect may be obtained on different body parts of the user. The weight may for example may larger at one end compared to the other, or the distribution of pressure points may be varied. Also different types/sizes of material may be used as the therapeutic material in one part of the sleeping bag compared to another part.

To be able to wash the sleeping bag, all components of the sleeping bag should be suitable for washing in a washing machine. Hence, in one embodiment, the therapeutic material is suitable for washing in a washing machine.

In one or more examples, the therapeutic material is substantially spherical objects. By using substantially spherical objects is obtained that the pressure upon the user's body is sporadic to avoid obstruction of the blood circulation. The spherical objects may be able to move easily in order not to apply pressure on the same point of the body.

In one or more examples, the substantially spherical objects have a shell of polymer, glass, metal or the like. The substantially spherical objects may be hollow, solid, or solid with a shell and a filling material such as fluids or solid material different from the shell material. The choice of spherical object may be a reflection of the user's age or therapeutic need.

The substantially spherical objects are perfectly spherical ball shaped objects.

In one or more examples, the substantially spherical objects are have a diameter in the range of 10 mm to 100 mm, such as 15 mm to 50 mm, or such as 18 mm to 45 mm, such as 20 mm to 40 mm. The diameter of the substantially spherical objects may be varied in order to accommodate the different size of users, e.g. a child or an adult. The diameter of the substantially spherical objects may also be varied in order to exert a pressure on the body of the user possible to to a lower or higher extend depending on the size of the objects. To obtain the calming effect it is essential that the pressure exerted on the body of the user is sporadic.

In one or more examples, the top section of the base part comprises a pillow arrangement for supporting the users head when using the sleeping bag. The pillow arrangement provides an increased thickness of at least a part of the top section. The pillow arrangement may comprise one or more filler materials for providing a pillow for the users head to rest on.

In one or more examples, the pillow arrangement is removably attached to the remainder of the sleeping bag by fastening means comprising one or more button(s), press stud(s), hook and loop fastener(s), zipper(s) or a combination thereof. Pillow may for example be arranged in the base part and be enclosed by a pouch comprised by the top section of the base part, and the pouch may be closed and opened by said fastening means. Alternatively, the pillow may be arranged onto an outermost layer of the base part, such as an inner surface, and attached to the base part by said fastening means. Advantageously, the pillow may then be sperated from the sleeping bag and thereby be replaced, used elsewhere and/or washed separately to the remainder of the sleeping bag etc.

In one or more examples, each of the edge surfaces of the cover part and the base part, comprises a feet edge part, a head edge part, a first body edge part, and a second body edge part. At least the second body edge parts are releasable connected for providing an easy user access to the sleeping bag, and wherein the head edge parts are not connected. The first body edge parts may be non-releasable connected. When the sleeping bag is opened, the non-releasable connection of the first body edges part, which may extend along one of the sides of the sleeping bag from top to feet part, will turn the sleeping bag into a blanket if the feet parts are releasable connected.

In one or more examples, at least the second body edge part of the base part are releasable connected to the second body edge part of the cover part by means of a connecting means, such as e.g. a zipper, button(s), press stud(s) and/or hook and loop fastener. The connecting means, e.g. a zipper, may extend only along one of the sides of the sleeping bag. Alternatively, the connecting means, e.g. zipper(s), button(s), press stud(s), hook and loop fastener(s) may extend along all edges of the cover part connected to the base part. Thus, the sleeping bag may openable all the way through thereby allowing it to be separated into two parts for easy handling, e.g. fitting into a regular sized washing machine.

In one or more examples, at least the first body edge part and the second body edge part of the base part is releasably connected to the first body edge part and second body edge part of the cover part, respectively, by means of a connecting arrangement, such as e.g. two or more zippers. This allows for a larger selectivity in how to open the sleeping bag.

In one or more examples, when the connecting means/connecting arrangement is/comprises zippers, each zipper may comprise at least one slider for opening and closing the zipper. In one or more examples, the zippers may each comprise a slider arranged at the bottom of the edges when in a closed configuration. Alternatively, at least one of the one or more zipper(s) may comprise a slider in each end of the zipper. This allows for the sleeping bag to be opened in a section along the zipper or along the collective extent of the zippers, with a closed configuration of the zipper(s) on each side of the opened section.

In one or more examples, the sleeping bag further comprises a feet part connected at peripheral edges to the feet edge parts of the base part and the cover part. Thereby an additional room for the feet of the user is provided.

In one or more examples, the bottom section of the cover part are separable from the middle section of the cover part by means of connecting means, e.g. a zipper. This allows for ventilation of the feet if needed.

In one or more examples, the base part comprises a top section positioned below the user's head and chest; a bottom section positioned below the user's feet and possibly the lower part of the user's leg; and a middle section positioned between the top section and bottom section. The bottom section of the base part together with the bottom section of cover part may be separable from the middle section of the base part and the middle section of the cover part by means of e.g. a zipper. The sleeping bag can thereby be used as a sleeping bag for users of different sizes, e.g. children, teenagers and adults. One sleeping bag may thereby be adjusted and formed into a smaller / larger size be removing / adding a feet part. The bottom section may be attached/detached by means of a zipper. Also, if the bottom section is detached, the middle sections may be connectable to form a new feet part in the sleeping bag with a reduced length adjusted for a smaller user, such as e.g. a child.

### Brief description of the drawings

Various examples are described hereinafter with reference to the figures. Like reference numerals refer to like elements throughout. Like elements will, thus, not be described in detail with respect to the description of each figure. It should also be noted that the figures are only intended to facilitate the description of the examples. They are not intended as an exhaustive description of the claimed invention or as a limitation on the scope of the claimed invention. In addition, an illustrated example needs not have all the aspects or advantages shown. An aspect or an advantage described in conjunction with a particular example is not necessarily limited to that example and can be practiced in any other examples even if not so illustrated, or if not so explicitly described.
Figure 1A-B show a base part of a sleeping bag and figures 1C-D show a cover part of a sleeping bag.
Figures 2A-B show a sleeping bag.
Figures 3A-B show a sleeping bag.
Figure 4 shows a cross sectional view of a part of a cover part of a sleeping bag.
Figure 5A-B show cross sectional views of two different base parts of a sleeping bag.
Figures 6 and 7 show two different cover parts for a sleeping bag.
Figure 8 shows a cross sectional view of a cover part of a sleeping bag.
Figure 9 and 10 show two different cover parts for a sleeping bag.
Figure 11 and 12 show two different cover parts for a sleeping bag.

### Description of examples

Exemplary examples will now be described more fully hereinafter with reference to the accompanying drawings. In this regard, the present examples may have different forms and should not be construed as being limited to the descriptions set forth herein. Accordingly, the examples are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

In the drawings, thicknesses of a plurality of layers and areas are illustrated in an enlarged manner for clarity and ease of description thereof. When a layer, area, element, or plate is referred to as being "on" another layer, area, element, or plate, it may be directly on the other layer, area, element, or plate, or intervening layers, areas, elements, or plates may be present therebetween. Conversely, when a layer, area, element, or plate is referred to as being "directly on" another layer, area, element, or plate, there are no intervening layers, areas, elements, or plates therebetween. Further when a layer, area, element, or plate is referred to as being "below" another layer, area, element, or plate, it may be directly below the other layer, area, element, or plate, or intervening layers, areas, elements, or plates may be present therebetween. Conversely, when a layer, area, element, or plate is referred to as being "directly below" another layer, area, element, or plate, there are no intervening layers, areas, elements, or plates therebetween.

The spatially relative terms "lower" or "bottom" and "upper" or "top", "below", "beneath", "less", "above", and the like, may be used herein for ease of description to describe the relationship between one element or component and another element or component as illustrated in the drawings. It will be understood that the spatially relative terms are intended to encompass different orientations of the device in use or operation, in addition to the orientation depicted in the drawings. For example, in the case where a device illustrated in the drawings is turned over, elements described as being on the "lower" side of other elements, or "below" or "beneath" another element would then be oriented on "upper" sides of the other elements, or "above" another element. Accordingly, the illustrative term "below" or "beneath" may include both the "lower" and "upper" orientation positions, depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below, and thus the spatially relative terms may be interpreted differently depending on the orientations described.

Throughout the specification, when an element is referred to as being "connected" to another element, the element is "directly connected" to the other element.

The terminology used herein is for the purpose of describing particular examples only and is not intended to be limiting. As used herein, the singular forms "a," "an," and "the" are intended to include the plural forms, including "at least one," unless the content clearly indicates otherwise. "At least one" is not to be construed as limiting "a" or "an." It will be further understood that the terms "comprises," "comprising," "includes" and/or "including," when used in this specification, specify the presence of stated features, integers, steps, operations, elements, and/or components, but do not preclude the presence or addition of one or more other features, integers, steps, operations, elements, components, and/or groups thereof.

It will be understood that, although the terms "first," "second," "third," and the like may be used herein to describe various elements, these elements should not be limited by these terms. These terms are only used to distinguish one element from another element. Thus, "a first element" discussed below could be termed "a second element" or "a third element," and "a second element" and "a third element" may be termed likewise without departing from the teachings herein.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

Exemplary examples are described herein with reference to cross section illustrations that are schematic illustrations of idealized examples, wherein like reference numerals refer to like elements throughout the specification. As such, variations from the shapes of the illustrations as a result, for example, of manufacturing techniques and/or tolerances, are to be expected. Thus, examples described herein should not be construed as limited to the particular shapes of regions as illustrated herein but are to include deviations in shapes that result, for example, from manufacturing. For example, a region illustrated or described as flat may have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims. Some of the parts which are not associated with the description may not be provided in order to specifically describe exemplary examples of the present disclosure.

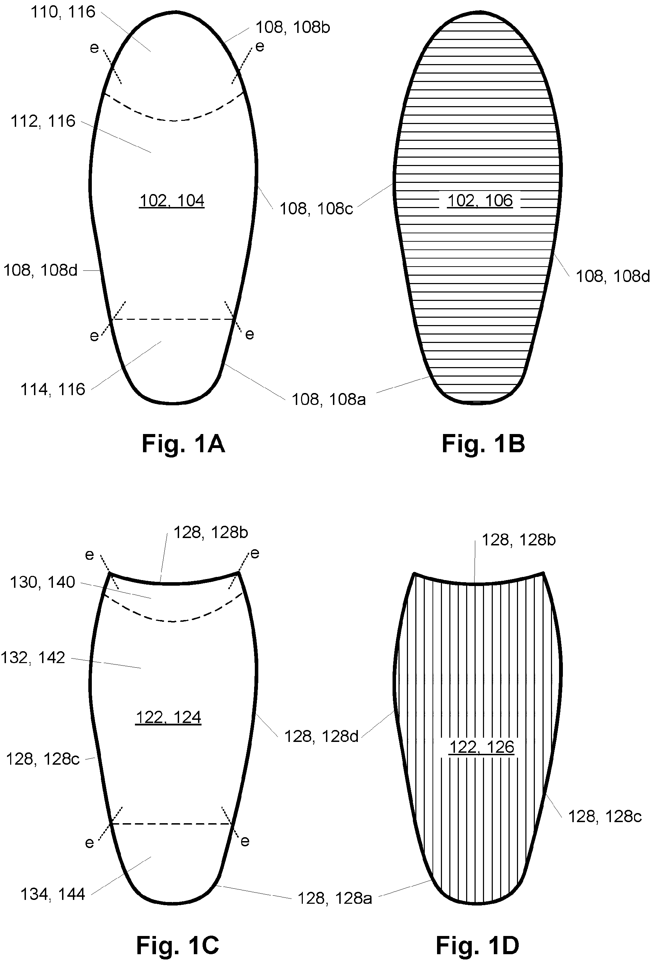

Figure 1A-B shows a base part 102 of a sleeping bag 100 on which a user rests. In figures 1C-D a cover part 122 of a sleeping bag 100 is shown. The cover part 122 is resting on top of the user when the user is positioned inside the sleeping bag. The cover and base parts normally have an elongated oval shape as often seen in regular sleeping bags.

Figure 1B shows the inner surface 106 of the base part 102 on which the user is positioned. The inner surface 126 of the cover part 122 is shown in figure 1D. Figure 1A and figure 1C shows the outer surface 104, 124 of the base part 102 and the cover part 122, respectively. The outer surfaces 104, 124 are not in direct contact with the user, when the user is positioned inside the sleep bag 100.

The horizontal lines shown on the inner surface 106 of the base part and the vertical lines shown on the inner surface 126 of the cover part 122 are only included to differentiate the inner and outer surfaces within the cover/base part and the two inner surfaces 106, 126. All surfaces 104, 106, 124, 126 may visually appear the same for a user. Alternatively, one or more of the surfaces may have a different colour or texture.

In figures 1A and 1C, dotes lines marks three sections in the base/cover part. The different sections are present in the entire base part 102, and the sections should also be envisioned to be present when viewing the cover/base part 102, 122 on the inner surface 106, 126 as shown in figure 1B and 1D. The sections in the base part 102 include a top section 110 positioned below the user's head and chest, a bottom section 114 positioned below the user's feet and possibly the lower part of the user's leg; and a middle section 112 positioned between the top section 110 and bottom section 112. The sections in the cover part 122 includes a top section 130 positioned on a user's chest, a bottom section 134 positioned on the user's feet and possibly the lower part of the user's leg, and a middle section 132 positioned between the top section 130 and the bottom section 134. The dotted lines through the cover/base part in figure 1A/C illustrates the transition between the sections. The transition could be found at locations further towards the bottom end or the top of the base/cover part.

The bottom section 114 of the base part 102 together with the bottom section 134 of cover part 122 may be separable from the middle section 112 of the base part 102 and the middle section 132 of the cover part 122 by means of e.g. a zipper.

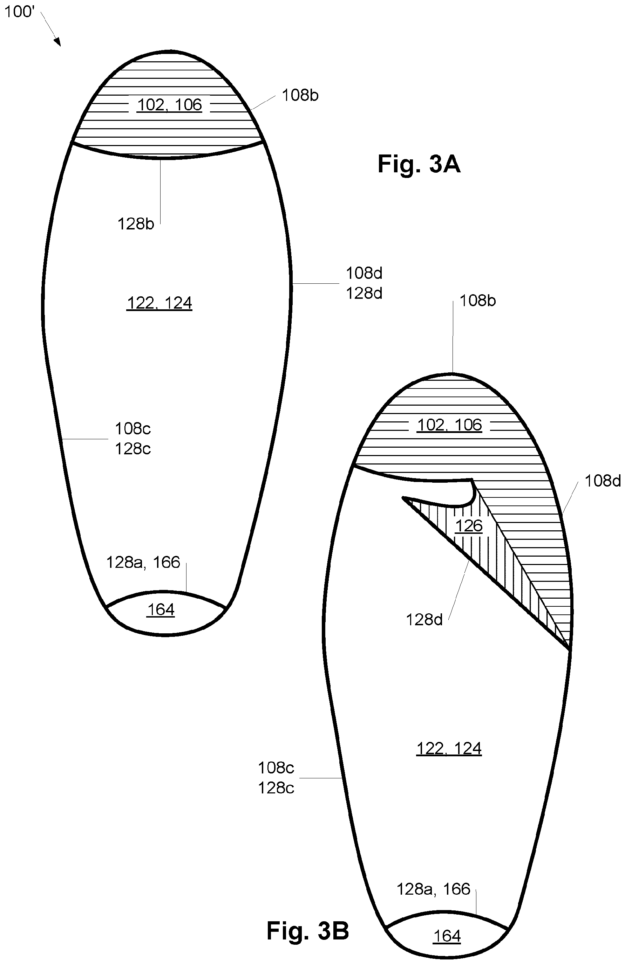

The base part 102 comprises an edge surface 108 connecting peripheral edges of the surfaces 104, 106. Likewise, the cover part 122 comprises an edge surface 128 connecting peripheral edges of the surfaces 124, 126. At least a part of the body edge surfaces 108, 128 are releasable connected for providing an easy user access to the sleeping bag. Each of the edge surfaces 108, 128 comprises a feet edge part 108a, 128a, a head edge part 108b, 128b, a first body edge part 108c, 128c, and a second body edge part 108d, 128d. The division into the different edge parts are marked with the small dotted lines with the letter e next to. At least the second body edge parts 108d, 128d are releasable connected for providing an easy user access to the sleeping bag. The feet edge parts 108a, 128a may be connected fixedly, realisably or to an additional feet part 164 as shown in figures 3A-B.

The head edge parts 108b, 128b are not connected as the user's head is protruding through the opening between the two head edge parts 108b, 118b when the user is positioned inside the sleeping bag 100. Normally, the head edge parts 108b, 128b will have different non-overlapping surfaces.

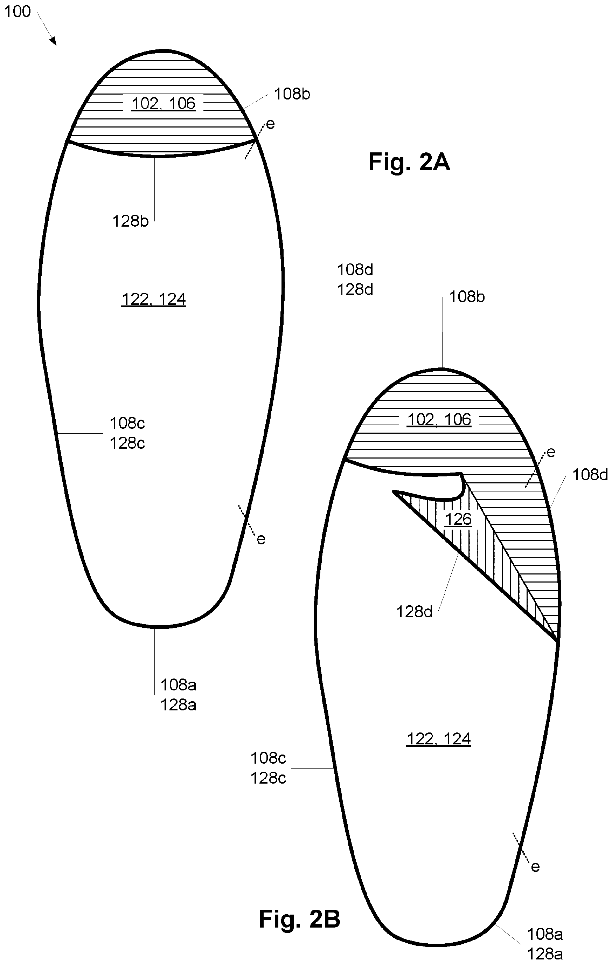

In figures 2A-B, a sleeping bag 100 with connected cover and base parts are shown. In figure 2A, the sleeping bag 100 is closed, whereas it is partly open in figure 2B. A slightly different version of the sleeping bag 100' is shown in figures 3A-B, where a feet part 164 is connected to the feet edge parts 108a, 118a at the peripheral edge 166 of the feet part 164. The feet part 164 will normally have an oval or round shape. Also, the inner and outer surface and general material choice for all layers of the feet part 164 may be similar to that of the base part 102, or the cover part 122 or sections 130, 132, 134 of the cover part 122. In one or more examples, the feet part 164 may include a therapeutic material according to one or more examples of the present invention.

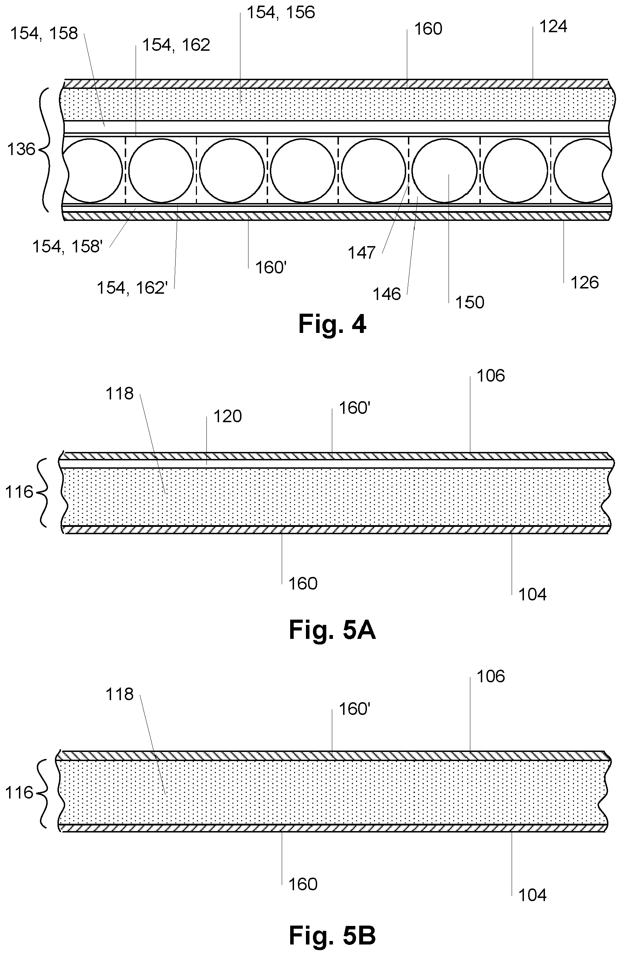

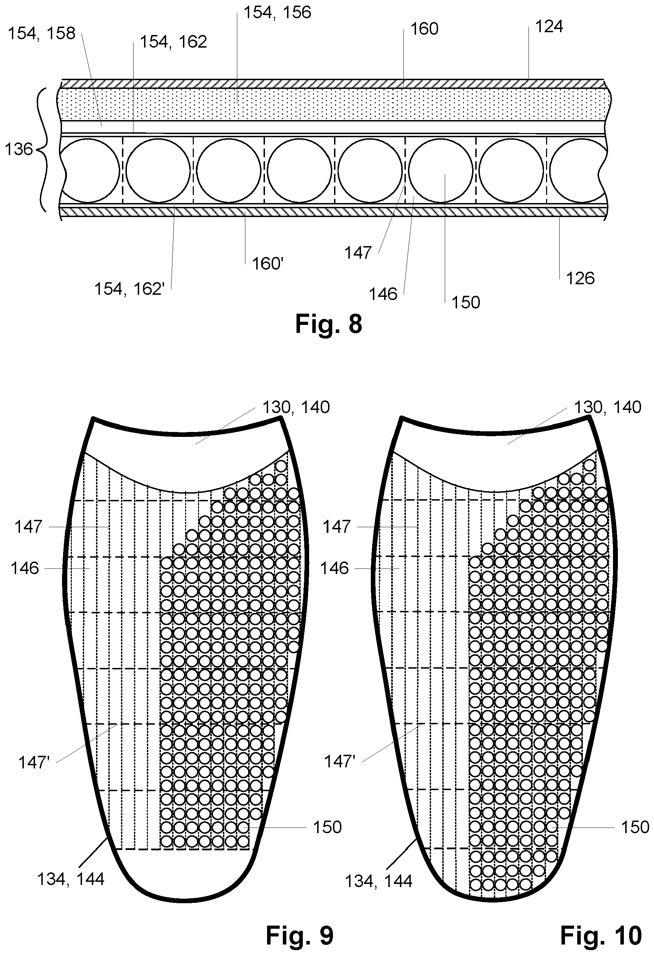

Between the outer and inner surfaces of the cover part 122, the base part 102 and the feet part 164 is a filling material 116, 136 as shown in figures 4, 5A-B and/or 8. Figure 4 and 8 shows a cross sectional view of two different versions of a cover part with therapeutic material 150 and figures 5A-B show a cross sectional view of the base part in two different versions.

The base filling 116 between the outer surface 104 and the inner surface 106 comprises at least one soft material layer 118. Similarly, cover filling 136 comprises one or more soft material layers 154.

The different cover part sections may have different cover fillings, and the filling in each section can therefore be described as a top section filling 140 in the top section 130 being positioned on a user's chest, a bottom section filling 144 in the bottom section 134 being positioned on the user's feet and possibly the lower part of the user's leg, and a middle section filling 142 in the middle section 132 being positioned between the top section 130 and the bottom section 134. This is illustrated in figures 1A-B.

The middle section filling 142 further comprises a therapeutic material 150 which adapt to the body of the user and exert a sporadic pressure on the body of the user through the inner surface 126. An example of such a filling with therapeutic spherical spheres 150 is shown in figure 4 and figure 8. The therapeutic material 150 is not limited to spherical spheres, but could be selected from the group of:
- substantially spherical objects; such as objects made from glass, polyethylene, polypropylene, polystyrene, silicone, seaweed, hemp, pineapple, corn, peas, beans (such as soy beans), lenses, or similar;
- fibre strips;
- granulate, such as granulate made from metal (such as steel), glass, polyethylene, polystyrene, polypropylene, silicone, seaweed, hemp, pineapple, corn, peas, beans (such as soy beans), lenses, or similar;
- substantially ring-formed objects; such as objects made from glass, polyethylene, polypropylene, polystyrene, silicone, seaweed, hemp, pineapple, corn, peas, beans (such as soy beans), lenses, or similar;
- chains, e.g. polymeric and/or metal objects connected to form a chain, and/or chains made from glass, polyethylene, polypropylene, polystyrene, silicone, seaweed, hemp, pineapple, corn, peas, beans (such as soy beans), lenses, or similar.

The sleeping bag 100 shown in the cross-sectional views in figures 4, 5A-B and 8 has a first layer of fabric 160 constituting the outer surfaces 104, 124, and a second layer of fabric 160' constituting the inner surfaces 106, 126. The soft material layers 118, 154 are positioned between the layers of fabric 160, 160' in both the cover part 122 and the base part 102.

The one or more soft material layers 118, 154 may comprise a first layer 120, 158 of nonwoven material and/or a second layer of nonwoven material 120', 158'. One or more of the soft material layers 154 may comprise temperature stabilising and/or insulating material. Such layer could e.g. be layer 120 and layer 158'.

The therapeutic material 150 is positioned inside an elastic material 162, 162' in the embodiment shown in figure 4. The elastic material 162, 162' is further positioned between the layers of e.g. non-woven material 158, 158'. The therapeutic material 150 is also positioned inside an elastic material 162, 162' in the embodiment shown in figure 8, however the soft material layers 154 only comprises a non-woven material 158 on one side of the therapeutic material 150, between one of the elastic material layers 162 and first layer of fabric 160.

The base part 102 shown in figure 5A comprises one layer of nonwoven material 120', whereas the nonwoven layers are absent in the base part shown in figure 5B.

As shown in figure 4, the therapeutic material 150 is positioned between the first layer 158 of e.g. nonwoven material and the second layer of e.g. nonwoven material 158'.

The one or more soft material layer 116, 156 could also comprise a hollow fibres. The hollow fibres layer 116, 156 is positioned between a layer 120', 158 of nonwoven material and a fabric layer 160, 160' in the shown figures. In figure 8 the hollow fibres layer 156 is positioned between the outer fabric layer 160 and the non-woven material 158.

In the cover part 122, the hollow fibres layer 156 is normally positioned between the outer fabric layer 160 and the first layer of non-woven material 158. In the base part 102, the hollow fibres layer 116 is normally positioned between the inner fabric layer 160' and the layer of non-woven material 120'.

The soft material layer(s) 116 could be a wadding layer 118 such as polymeric wadding, such as polyester wadding. Hollow fibres, such as hollow polymeric fibres, such as hollow polyester fibres may be used as an alternative to the wadding. The wadding layer 116 is normally positioned between the inner fabric layer 160' and the layer of non-woven material 120'. The wadding layer 118 could also be positioned between the two layers of nonwoven material (not shown in the figure).

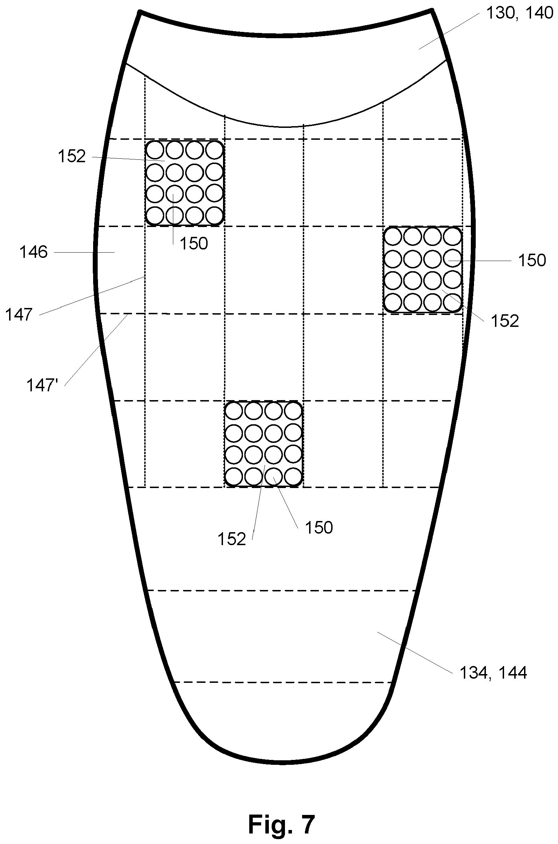

The therapeutic material 150 can be positioned in a plurality of bags 152 as shown in figure 7. Different bags 152 may have different weights.

As shown in figures 4, 6, 7 and 9-12, a plurality of channels 146 is formed between the outer surface 124 and inner surface 126 of at least the middle section 132 in the cover part, by one or more joints 147, 147', wherein the channels 146 are filled with the therapeutic material.

In figures 9 and 10, a plurality of channels 146 are formed in both the middle section 132 and at least in a part of the bottom section 134. In figure 9, a plurality of channels 146 filled with therapeutic material are extending from an border between the middle section 132 and the bottom section 134 and terminating at a position towards a distal edge surface of the bottom section 134, leaving a part of the bottom section 134, near the distal edge surface of the bottom section 134, devoid of any channels 146 of therapeutic material. In an embodiment, the bottom section devoid of therapeutic material, may be arranged in an area configured to be typically covering the feet of the user, when the sleeping bag is in use. E.g. the channels 146 filled with therapeutic material may extend from the border between the middle section 132 and the bottom section 134 and across approximately 30-70%, or such as at least 50%, of the longitudinal extent of the bottom section, wherein the longitudinal extent is measured from the border to the distal edge surface of the bottom section along a longitudinal extent of the sleeping bag.

In figure 10, a plurality of channels 146 filled with therapeutic material is provided across substantially the entire extent of the bottom section 134 in the cover part.

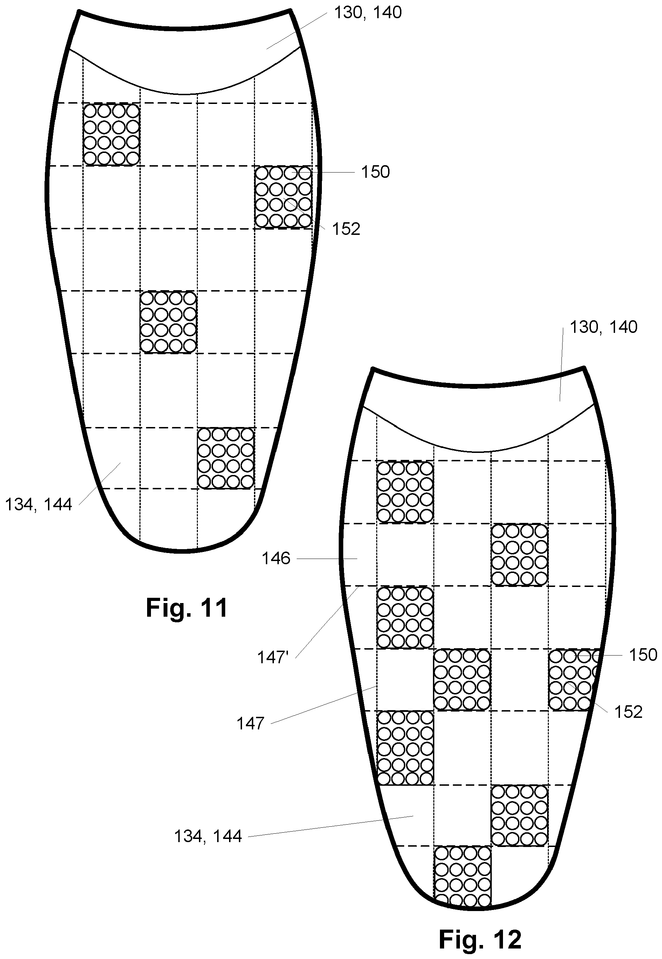

In figures 11 and 12, a plurality of channels 146 are formed in both the middle section 132 and at least in a part of the bottom section 134. In figure 11, a plurality of channels 146 filled with a plurality of bags 152 with therapeutic material 150 are extending from an border between the middle section 132 and the bottom section 134 and terminating at a position towards a distal edge surface of the bottom section 134, leaving a part of the bottom section 134, near the distal edge surface of the bottom section 134, devoid of any channels 146 of therapeutic material. In an embodiment, the bottom section devoid of therapeutic material, may be arranged in an area configured to be typically covering the feet of the user, when the sleeping bag is in use. E.g. the channels 146 filled with bags 152 with therapeutic material may extend from the border between the middle section 132 and the bottom section 134 and across approximately 30-70%, or such as at least 50%, of the longitudinal extent of the bottom section, wherein the longitudinal extent is measured from the border to the distal edge surface of the bottom section along a longitudinal extent of the sleeping bag.

In figure 12, a plurality of channels 146 filled with bags 152 containing therapeutic material 150 is provided across substantially the entire extent of the bottom section 134 in the cover part. The different bags 152 may have different weights.

The joints 147, 147' may be made in the elastic material surrounding/covering the therapeutic material 150 in the embodiment shown in figure 4 and 8. The joints 147, 147' could be a stitching.

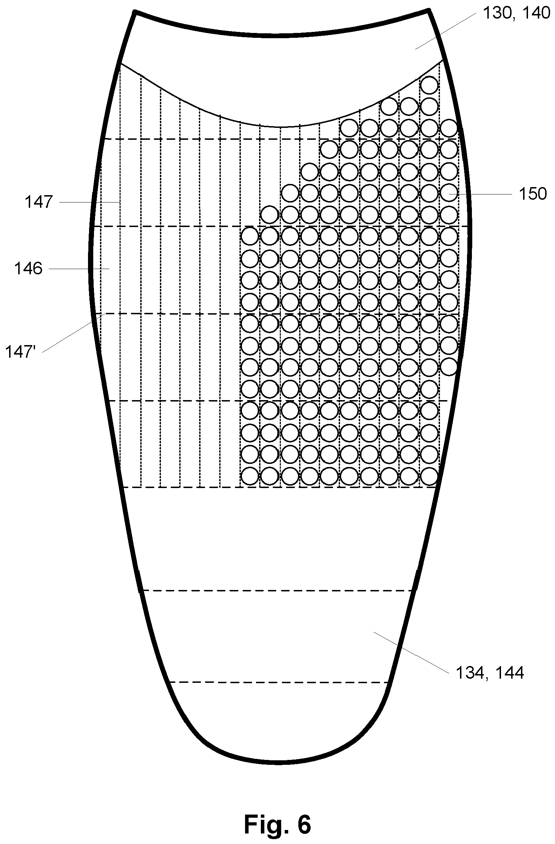

The channels 146 run in the length-wise and/or width-wise direction of the sleeping bag 100 in the embodiments shown in figures 6-7 and 9-12. Other directional setups could also be imagined. The channels 146 shown in figures 6-7 and 9-12 are evenly distributed.

In the embodiments shown in figures 6-7 and 9-12, the substantially spherical objects constitute the therapeutic material 150. The channels 146 in figures 6-7 and 9-12 are of a size that does not allow one substantially spherical object / one bag to pass another substantially spherical object / bag within the channel 146. This also applies if different materials are used as therapeutic material 150. In figures 6-7 and 9-12, only some channels are shown with therapeutic material 150 to simplify the figures.

The therapeutic material 150 in the middle section filling 142 may be positioned in the plurality of bags 152 whereas the therapeutic material 150 in the bottom section filling 144 could be positioned directly in the plurality of channels 146. Such an embodiment is a combination of the two embodiments shown in figures 6 and 7.

The invention is further described in the following items.
1. A sleeping bag (100) for therapeutic treatment, the sleeping bag (100) comprising a base part (102) on which a user rests, and a cover part (122) covering the user's body when positioned in the sleeping bag;
   wherein the base part (102) comprises:
   ∘ an outer surface (104);
   ∘ an inner surface (106);
   ∘ an edge surface (108) connecting peripheral edges of the surfaces (104, 106);
   ∘ a base filling (116) between the outer surface (104) and the inner surface (106);
   wherein the cover part (122) comprises:
   ∘ an outer surface (124);
   ∘ an inner surface (126);
   ∘ an edge surface (128) connecting peripheral edges of the surfaces (124, 126), wherein at least a part of the body edge surfaces (108, 128) are releasable connected for providing an easy user access to the sleeping bag;
   ∘ a number of cover part sections with cover filling (136) between the outer surface (124) and the inner surface (126), the sections and cover filling (136) including:
      - a top section (130) with a top section filling (140), the top section (130) being positioned on a user's chest;
      - a bottom section (134) with a bottom section filling (144), the bottom section (134) being positioned on the user's feet and possibly the lower part of the user's leg; and
      - a middle section (132) with a middle section filling (142), the middle section (132) being positioned between the top section (130) and the bottom section (134);
   wherein the base filling (116) and the one or more cover filings (136) comprises one or more soft material layers (118, 154);
   wherein the middle section filling (142) further comprises a therapeutic material (150) which adapt to the body of the user and exert a sporadic pressure on the body of the user through the inner surface (126).
2. The sleeping bag according to any preceding item, wherein the base filling (116) does not comprise the therapeutic material (150).
3. The sleeping bag according to any preceding item, wherein the top section filling (140) does not comprise the therapeutic material (150).
4. The sleeping bag according to any of the items 1-2, wherein the top section filling (140) further comprises the therapeutic material (150).
5. The sleeping bag according to any preceding item, wherein the bottom section filling (144) further comprises the therapeutic material (150).
6. The sleeping bag according to any preceding item, wherein the one or more soft material layers (118, 154) are selected from the group of:
   ∘ polymeric fibres, such as polyester fibres;
   ∘ hollow fibres, such as hollow polymeric fibres, such as hollow polyester fibres;
   ∘ plant fibres, such as fibres from corn, bamboo, viscose, jute, pineapple, seaweed, kapok, hemp;
   ∘ cupro fibres;
   ∘ wadding, such as polymeric wadding, such as polyester wadding;
   ∘ down.
7. The sleeping bag according to any preceding item, wherein the sleeping bag comprises a first layer of fabric (160) constituting the outer surfaces (104, 124), and a second layer of fabric (106') constituting the inner surfaces (106, 126), wherein the soft material layer(s) (154) are positioned between the layers of fabric (160, 160') in both the cover part (122) and the base part (102).
8. The sleeping bag according to any preceding item, wherein the one or more soft material layers (118, 154) comprises one or more layers of temperature stabilising and/or insulating material.
9. The sleeping bag according to any preceding item, wherein the therapeutic material (150) is positioned inside an elastic material (162, 162').
10. The sleeping bag according to any preceding item, wherein the one or more soft material layers (118, 154) comprises a first layer (120, 158) of nonwoven material and/or a second layer of nonwoven material (120', 158').
11. The sleeping bag according to item 10, wherein the elastic material (162, 162') is positioned between the layers of non-woven material (158, 158').
12. The sleeping bag according to item 10, wherein the cover part comprises only one layer of nonwoven material (158).
13. The sleeping bag according to any of the items 10-11, wherein the base part (102) only comprises one layer of nonwoven material (120').
14. The sleeping bag according to any of the items 10-13, wherein in the filling sections comprising the therapeutic material (150), the therapeutic material (150) is positioned between the first layer (158) of nonwoven material and the second layer of nonwoven material (158').
15. The sleeping bag according to any of the items 10-14, wherein in the filling sections comprising the therapeutic material (150), the thereapeutic material (150) is positioned between the first layer (158) of nonwoven material and the inner surface (124).
16. The sleeping bag according to any of the items 10-15, wherein in the filling sections comprising the therapeutic material (150), the thereapeutic material (150) is positioned between the first layer (158) of nonwoven material and the outer surface (126).
17. The sleeping bag according to any preceding item, wherein the one or more soft material layers (154) comprises a hollow fibres layer (116, 156) of hollow fibres, such as hollow polymeric fibres, such as hollow polyester fibres.
18. The sleeping bag according to item 17, wherein the hollow fibres layer (116, 156) is positioned between a layer (120', 158) of nonwoven material and a fabric layer (160, 160').
19. The sleeping bag according to any preceding item, wherein the one or more soft material layers (116) comprises a wadding layer (118) such as polymeric wadding, such as polyester wadding.
20. The sleeping bag according to item 19, wherein the wadding layer (118) is positioned between the two layers (120, 120') of nonwoven material.
21. The sleeping bag according to any preceding item, wherein the therapeutic material (150) is selected from the group of:
   ∘ substantially spherical objects; such as objects made from metal (such as steel), glass, polyethylene, polypropylene, polystyrene, silicone, seaweed, hemp, pineapple, corn, peas, beans (such as soy beans), lenses, or similar;
   ∘ fibre strips;
   ∘ granulate, such as granulate made from metal (such as steel), glass, polyethylene, polypropylene, polystyrene, silicone, seaweed, hemp, pineapple, corn, peas, beans (such as soy beans), lenses, or similar;
   ∘ substantially ring-formed objects; such as objects made from metal (such as steel), glass, polyethylene, polypropylene, polystyrene, silicone, seaweed, hemp, pineapple, corn, peas, beans (such as soy beans), lenses, or similar;
   ∘ chains, e.g. polymeric and/or metal objects connected to form a chain, and/or chains made from metal (such as steel), glass, polyethylene, polypropylene, polystyrene, silicone, seaweed, hemp, pineapple, corn, peas, beans (such as soy beans), lenses, or similar.
22. The sleeping bag according to any preceding item wherein the therapeutic material (150) is positioned in a plurality of bags (152).
23. The sleeping bag according to item 22, wherein each bag has a weight, wherein the weight of a first bag is different from the weight of a second bag.
24. The sleeping bag according to any preceding item, wherein a plurality of channels (146) is formed between the outer surface (124) and inner surface (126) of at least the middle section (132) in the cover part, by one or more joints (147, 147'), wherein the channels (146) are filled with the therapeutic material.
25. The sleeping bag according to item 24, wherein the channels (146) run in the length-wise and/or width-wise direction of the sleeping bag (100).
26. The sleeping bag according to any of the items 24-25, wherein the one or more the joints (147, 147') is a stitching.
27. The sleeping bag according to any of the items 24-26, wherein the channels (146) are evenly distributed.
28. The sleeping bag according to any of the items 24-27, wherein the channels (146) are formed in the elastic material (162, 162') covering the therapeutic material (150).
29. The sleeping bag according to any of the items 24-28, wherein each of the channels (146) are of a size that does not allow one substantially spherical object / one substantially ring-formed object / one bag to pass another substantially spherical object / substantially ring-formed object / bag within the channel (146).
30. The sleeping bag according to any of the items 22-29, wherein the therapeutic material (150) in the middle section filling (142) is positioned in the plurality of bags (152) and the therapeutic material (150) in the bottom section filling (144) is positioned directly in the plurality of channels (146).
31. The sleeping bag according to any preceding item, wherein the therapeutic material (150) is substantially spherical objects.
32. The sleeping bag according to any of the items 31, wherein the substantially spherical objects have a shell of polymer, glass, metal or the like.
33. The sleeping bag according to item 32, wherein the substantially spherical objects are hollow, solid, or solid with a shell and a filling material such as fluids or solid material different from the shell material.
34. The sleeping bag according to any of the items 31-33, wherein the substantially spherical objects are perfectly spherical ball shaped objects.
35. The sleeping bag according to any of the items 31-34, wherein the substantially spherical objects are have a diameter in the range of 10 mm to 100 mm, such as 15 mm to 50 mm, or such as 18 mm to 45 mm, such as 20 mm to 40 mm.
36. The sleeping bag according to any preceding item, wherein each of the edge surfaces (108, 128) comprises a feet edge part (108a, 128a), a head edge part (108b, 128b), a first body edge part (108c, 128c), and a second body edge part (108d, 128d), wherein at least the second body edge parts (108d, 128d) are releasable connected for providing an easy user access to the sleeping bag, and wherein the head edge parts (108b, 128b) are not connected.
37. The sleeping bag according to item 36, wherein the first body edge parts (108c, 128c) are non-releasable connected.
38. The sleeping bag according to any of the items 36-37, wherein at least the second body edge part (108d) of the base part (102) are releasable connected to the second body edge part (128d) of the cover part (122) by means of connecting means, such as e.g. a zipper.
39. The sleeping bag according to any of the items 36-38, wherein at least the first body edge part (108d) of the base part (102) are releasable connected to the first body edge part (128d) of the cover part (122) by means of connecting means, such as e.g. a zipper.
40. The sleeping bag according to any of the items 36-39, wherein the first and second body edge part (108d) of the base part (102) are releasable connected to the first and second body edge part (128d) of the cover part (122) by means of connecting means, such as e.g. a zipper.
41. The sleeping bag according to any of the items 36-40, wherein the first and second body edge part (108d) of the base part (102) are releasable connected to the first and second body edge part (128d) of the cover part (122) by means of a connecting arrangement, such as e.g. a zipper arrangement comprising at least two zippers.
42. The sleeping bag according to any of the items 36-41, wherein when the connecting means/connecting arrangement comprises zippers, each zipper comprises a slider for opening and closing the zipper, wherein the slider is arranged at the bottom of the sleeping bag when the zipper is closed.
43. The sleeping bag according to any of the items 36-42, wherein when the connecting means/connecting arrangement comprises zippers, each zipper comprises at least two sliders for opening and closing the zipper.
44. The sleeping bag according to any of the items 36-38, wherein the sleeping bag (100) further comprises a feet part (164) connected at peripheral edges (166) to the feet edge parts (108a, 128a) of the base part (102) and the cover part (122).
45. The sleeping bag according to any of the preceding items, wherein the top section of the base part further comprises a pillow arrangement, for providing a pillow for the head of the user.
46. The sleeping bag according to claim 45, wherein the pillow arrangement is removably attached to the remainder of the sleeping bag by fastening means comprising one or more button(s), press stud(s), hook and loop fastener(s), zipper(s) or a combination thereof.
47. The sleeping bag according to any preceding item, wherein the bottom section (134) of the cover part (122) are separable from the middle section (132) of the cover part (122) by means of connecting means, such as e.g. a zipper.
48. The sleeping bag according to any preceding item, wherein the base part (102) comprises:
   ∘ a top section (110) positioned below the user's head and chest;
   ∘ a bottom section (114) positioned below the user's feet and possibly the lower part of the user's leg; and
   ∘ a middle section (112) positioned between the top section (110) and bottom section (112);
   wherein the bottom section (114) of the base part (102) together with the bottom section (134) of cover part (122) are separable from the middle section (112) of the base part (102) and the middle section (132) of the cover part (122) by means of e.g. a zipper.

### References

- 100, 100': sleeping bag
- 102: base part
- 104: outer surface of the base part
- 106: inner surface of the base part
- 108: edge surface of the base part
- 108a: feet edge part of the base part
- 108b: head edge part of the base part
- 108c: first body edge part of the base part
- 108d: second body edge part of the base part
- 110: top section of the base part
- 112: middle section of the base part
- 114: bottom section of the base part
- 116: base filling
- 118: soft material
- 120: nonwoven material
- 122: cover part
- 124: outer surface of the cover part
- 126: inner surface of the cover part
- 128: edge surface of the cover part
- 128a: feet edge part of the cover part
- 128b: head edge part of the cover part
- 128c: first body edge part of the cover part
- 128d: second body edge part of the cover part
- 130: top section of the cover part
- 132: middle section of the cover part
- 134: bottom section of the cover part
- 136: cover filling
- 140: top section filling
- 142: middle section filling
- 144: bottom section filling
- 146: channel
- 147, 147': joint
- 150: therapeutic material, e.g. substantially spherical objects
- 152: bag filled with therapeutic material
- 154: soft material
- 156: hollow fibres material layer
- 158, 158': nonwoven material
- 160, 160': fabric
- 162, 162': elastic material
- 164: feet part
- 166: peripheral edges

## Claims

1. A sleeping bag (100) for therapeutic treatment, the sleeping bag (100) comprising a base part (102) on which a user rests, and a cover part (122) covering the user's body when positioned in the sleeping bag;
wherein the base part (102) comprises:
∘ an outer surface (104);
∘ an inner surface (106);
∘ an edge surface (108) connecting peripheral edges of the surfaces (104, 106);
∘ a base filling (116) between the outer surface (104) and the inner surface (106);
wherein the cover part (122) comprises:
∘ an outer surface (124);
∘ an inner surface (126);
∘ an edge surface (128) connecting peripheral edges of the surfaces (124, 126), wherein at least a part of the body edge surfaces (108, 128) are releasable connected for providing an easy user access to the sleeping bag;
∘ a number of cover part sections with cover filling (136) between the outer surface (124) and the inner surface (126), the sections and cover filling (136) including:
• a top section (130) with a top section filling (140), the top section (130) being positioned on a user's chest;
• a bottom section (134) with a bottom section filling (144), the bottom section (134) being positioned on the user's feet and possibly the lower part of the user's leg; and
• a middle section (132) with a middle section filling (142), the middle section (132) being positioned between the top section (130) and the bottom section (134);
wherein the base filling (116) and the one or more cover filings (136) comprises one or more soft material layers (118, 154);
wherein the middle section filling (142) further comprises a therapeutic material (150) positioned between the inner surface (126) and the outer surface (124) and wherein the therapeutic material (150) is arranged to adapt to the body of the user and exert a sporadic pressure on the body of the user through the inner surface (126).

2. The sleeping bag according to any preceding claim, wherein the base filling (116) and the top section filling (140) does not comprise the therapeutic material (150).

3. The sleeping bag according to any preceding claim, wherein the bottom section filling (144) further comprises the therapeutic material (150).

4. The sleeping bag according to any preceding claim, wherein the one or more soft material layers (118, 154) are selected from the group of:
∘ polymeric fibres, such as polyester fibres;
∘ hollow fibres, such as hollow polymeric fibres, such as hollow polyester fibres;
∘ plant fibres, such as fibres from corn, bamboo, viscose, jute, pineapple, seaweed, kapok, hemp;
∘ cupro fibres;
∘ wadding, such as polymeric wadding, such as polyester wadding;
∘ down.

5. The sleeping bag according to any preceding claim, wherein the one or more soft material layers (118, 154) comprises one or more layers of temperature stabilising and/or insulating material.

6. The sleeping bag according to any preceding claim, wherein the therapeutic material (150) is positioned inside an elastic material (162, 162').

7. The sleeping bag according to any preceding claim, wherein the one or more soft material layers (154) comprises:
• a hollow fibres layer (116, 156) of hollow fibres, such as hollow polymeric fibres, such as hollow polyester fibres, and/or
• a wadding layer (118, 156) such as polymeric wadding, such as polyester wadding and/or
• a first layer (120, 158) of nonwoven material and/or a second layer of nonwoven material (120', 158').

8. The sleeping bag according to claim 7, wherein in the filling sections comprising the therapeutic material (150), the therapeutic material (150) is positioned between the first layer (158) of nonwoven material and the second layer of nonwoven material (158').

9. The sleeping bag according to claim 7, wherein in the filling sections comprising the therapeutic material (150), the therapeutic material (150) is positioned between the first layer (158) of nonwoven material and the inner surface (126).

10. The sleeping bag according to claim 9, wherein the soft material layer(s) between the therapeutic material (150) and the inner surface (126) is/are devoid of a non-woven material layer (158').

11. The sleeping bag according to any preceding claim, wherein the therapeutic material (150) is selected from the group of:
∘ substantially spherical objects; such as objects made from metal (such as steel), glass, polyethylene, polypropylene, polystyrene, silicone, seaweed, hemp, pineapple, corn, peas, beans (such as soy beans), lenses, or similar;
∘ fibre strips;
∘ granulate, such as granulate made from metal (such as steel), glass, polyethylene, polypropylene, polystyrene, silicone, seaweed, hemp, pineapple, corn, peas, beans (such as soy beans), lenses, or similar;
∘ substantially ring-formed objects; such as objects made from metal (such as steel), glass, polyethylene, polypropylene, polystyrene, silicone, seaweed, hemp, pineapple, corn, peas, beans (such as soy beans), lenses, or similar;
∘ chains, e.g. polymeric and/or metal objects connected to form a chain and/or chains made from metal (such as steel), glass, polyethylene, polypropylene, polystyrene, silicone, seaweed, hemp, pineapple, corn, peas, beans (such as soy beans), lenses, or similar.

12. The sleeping bag according to any preceding claim wherein the therapeutic material (150) is positioned in a plurality of bags (152).

13. The sleeping bag according to any preceding claim, wherein a plurality of channels (146) is formed between the outer surface (124) and inner surface (126) of at least the middle section (132) in the cover part, by one or more joints (147, 147'), wherein the channels (146) are filled with the therapeutic material.

14. The sleeping bag according to claim 13, wherein the channels (146) are formed in the elastic material (162, 162') covering the therapeutic material (150), and wherein each of the channels (146) are of a size that does not allow one substantially spherical object / one substantially ring-formed object / one bag to pass another substantially spherical object / substantially ring-formed object / bag within the channel (146).

15. The sleeping bag according to any preceding claim, wherein the therapeutic material (150) is substantially spherical objects, such as substantially spherical objects having a shell of polymer, glass, metal or the like.

16. The sleeping bag according to claim 15, wherein the substantially spherical objects are have a diameter in the range of 10 mm to 100 mm, such as 15 mm to 50 mm, or such as 18 mm to 45 mm, such as 20 mm to 40 mm.

17. The sleeping bag according to any preceding claim, wherein the sleeping bag (100) further comprises a feet part (164) connected at peripheral edges (166) to feet edge parts (108a, 128a) of the base part (102) and the cover part (122).
